# EUROPEAN PATENT SPECIFICATION

(11) **EP 2 113 225 B1**
(45) Date of publication and mention of the grant of the patent: **10.11.2010**
(21) Application number: 09160154.2
(22) Date of filing: 30.03.2001
(51) Int. Cl.: A61F 2/06

(54) **Delivery catheter for bifurcated graft**
Abgabekatheter für gabelförmiges Transplantat
Cathéter pour la mise en place d'une endoprothèse bifurquée

(30) Priority: 30.03.2000 US 538997
(43) Date of publication of application: 04.11.2009
(62) Divisional of application: 01926575.0
(73) Proprietor: Teramed Corporation, Maple Grove, MN 55311 (US)
(72) Inventor: Petrick, Timothy, Brooklyn Park, MN 55443 (US); Willard, Steven, Minneapolis, MN 55412 (US)
(74) Representative: Brown, George Laurence

(56) References cited:
- WO-A-97/18006
- WO-A-98/23241
- WO-A-98/42276

## Description

### FIELD OF THE INVENTION

This invention relates to an apparatus for deploying a graft at an area of vessel bifurcation. In particular, this invention relates to an apparatus for deployment of a graft at the bifurcation of the common iliac artery for use in repairing abdominal aortic aneurysms.

### BACKGROUND OF THE INVENTION

Aortic aneurysms represent a significant medical problem for the general population. Aneurysms within the aorta presently affect between two and seven percent of the general population and the rate of incidence appears to be increasing. This form of vascular disease is characterized by a degradation in the arterial wall in which the wall weakens and balloons outward by thinning. If untreated, the aneurysm can rupture resulting in death within a short time.

The traditional treatment for patients with an abdominal aortic aneurysm is surgical repair. This is an extensive operation involving transperitoneal or retroperitoneal dissection of the aorta and replacement of the aneurysm with an artificial artery known as a prosthetic graft. This procedure requires exposure of the aorta through an abdominal incision extending from the lower border from the breast bone down to the pubic bone. The aorta is clamped both above and below the aneurysm so that the aneurysm can be opened and the prosthetic graft of approximately the same size as the aorta can be sutured in place. Blood flow is then re-established through the prosthetic graft. The operation requires a general anesthesia with a breathing tube, extensive intensive care unit monitoring in the immediate post-operative period along with blood transfusions and stomach and bladder tubes. All of this imposes stress on the cardiovascular system. This is a high-risk surgical procedure with well-recognized morbidity and mortality.

More recently, significantly less invasive clinical approaches to aneurysm repair known as endovascular grafting have been proposed. (See, Parodi, J. C., et al. "Transfemoral Intraluminal Graft Implantation for Abdominal Aortic Aneurysms," 5 Annals of Vascular Surgery, 491 (1991)). Endovascular grafting involves the transluminal placement of a prosthetic arterial graft in the endoluminal position (within the lumen of the artery). By this method, the graft is attached to the internal surface of an arterial wall by means of attachment devices such as expandable stents, one above the aneurysm and a second below the aneurysm.

It is not uncommon for abdominal aortic aneurysms to extend to the aortic bifurcation or even into the common iliac arteries. When the aneurysm extends into the common iliac arteries it is necessary that the graft system used to repair the aneurysm extend into the common iliac arteries past the aneurysm. This requires that there be enough space between the aneurysm and the common iliac bifurcation so that the graft can properly seat. By "seating" it is meant that the graft is somehow fixed to the non-aneurysmal vasculature. However, in a significant number of patients the aneurysm extends into the common iliac arteries on one or both sides such that there is not enough room to seat the graft without at least partially blocking the internal iliac artery. Such a situation occurs in so-called Class D or E aneurysms. The internal iliac artery is a significant vessel which supplies blood to the pelvic region. Blockage of the vessel can result in undesirable consequences for the patient. For this reason, patients in this category are often excluded from the less expensive and less traumatic endovascular repair and must instead undergo the invasive surgical procedure described above.

A prosthetic graft that allows endoluminal reconstruction of the common, external, and internal iliac bifurcation is disclosed in commonly assigned, co-pending application for "Endovascular Graft System", U.S.S.N. 09/454,038, filed December 3, 1999. However, need still exists for an improved system that will deliver and deploy such a graft to the iliac bifurcation.

WO-A-98/42276 relates to a stent graft delivery system. In one embodiment a graft delivery component for a bifurcated graft includes an elastomeric sleeve near the distal end. This grips a guidewire which is threaded through a leg of a graft in a doubled-over manner. A snare is used to capture the end of the guidewire and pull it free of the sleeve.

### SUMMARY OF THE INVENTION

According to the present invention, there is provided a delivery catheter as defined in appended claim 1. A delivery catheter has a distal end for insertion in a patient's vasculature for delivering a prosthesis comprising: an inner catheter over which the prosthesis is loaded; a retrograde guide wire has a main core and a retrograde core, the retrograde core having a first end joined to the main core and a second end; and a handle at a proximal end of the delivery catheter having a first control element for advancing the main core in a distal direction or retracting the main core in a proximal direction, the retrograde guide wire being configured such that retraction of the main core causes the second end of the retrograde core to move away from the main core. The delivery catheter may further comprise a retractable outer sheath defining a lumen through which the inner catheter extends and further define a space between the inner catheter and the sheath configured to contain the prosthesis. The handle may further comprise a second control element for retracting the outer sheath.

The control element may be adapted to retract the retrograde guide wire. The bifurcated prosthesis may be provided with a first engaging member adjacent the second end and the retrograde core further comprises a second engaging member, the second engaging member being configured to engage the first engaging member when the retrograde guide wire is retracted such that the second end of the side branch is moved away from the first end of the side branch.

The present invention may be used in a method for delivering a bifurcated prosthesis at the bifurcation of a first vessel into second and third vessels in a patient's vasculature using a single delivery catheter, the bifurcated prosthesis having a main branch with first and second ends adapted for placement in the first and second vessels, respectively, and a side branch defining a lumen and having an end adapted for placement in the third vessel, the bifurcated prosthesis capable of expansion from a first delivery configuration to a second deployed configuration, the method comprising : providing a delivery catheter including an inner catheter upon which the bifurcated prosthesis is positioned in its delivery configuration, the delivery catheter further having a retrograde guide wire having a main core and retrograde core, the retrograde core having a first end joined to the main core and a second end, the retrograde core being adapted to extend through the lumen of the side branch; advancing the delivery catheter through the patient's vasculature to a position where the first end of the main branch lies within the first vessel and the second end lies within the second vessel; manipulating the retrograde guide wire to cause the retrograde core to be extended into the third vessel; advancing the side branch into the third vessel; expanding the bifurcated prosthesis to the deployed configuration so that the first end is deployed in the first vessel, the second end is deployed in the second vessel, and the end of the side branch is deployed in the third vessel; and removing the delivery catheter from the patient's vasculature.

The invention may also be used in a method for repairing an abdominal aneurysm in an aorta which branches into first and second common iliac arteries using a main graft section having a lumen and first and second ends and a bifurcated graft section having a main branch having a lumen and first and second ends and a side branch having a lumen and an end, the first common iliac artery branching into an internal iliac artery and an external iliac artery, the method comprising: providing a guide wire; advancing the guide wire through the first common iliac artery to a desired location in the aorta above the aneurysm; providing a first delivery catheter containing the main graft section; advancing the first delivery catheter over the guide wire; deploying the main graft section within the aorta such that the first end is above the aneurysm and the second end extends towards the first common iliac artery; removing the first delivery catheter; providing a second delivery catheter containing the bifurcated graft section, the second delivery catheter having a retrograde guide wire including a retrograde core extending through the lumen of the side branch; advancing the second delivery catheter over the guide wire to a desired location in the first common iliac artery; manipulating the retrograde guide wire to extend the retrograde core into the internal iliac artery; retracting the delivery catheter in a proximal direction to extend the side branch into the internal iliac artery; deploying the bifurcated ; section from a first contracted delivery configuration to a second expanded deployed configuration such that the first end of the main branch is within the lumen of the main graft section adjacent the second end thereof, the second end of the main branch is within the external iliac artery and the end of the side branch is within the internal iliac artery; and withdrawing the second delivery catheter.

The step of manipulating the retrograde guide wire may include retracting the retrograde guide wire in a proximal direction. The step of deploying the bifurcated graft section may include deploying the main branch prior to deploying the side branch and wherein the method further includes applying tension to the side branch in the direction of the internal iliac artery to further extend the side branch into the internal iliac artery. The method may further include applying tension to the side branch in the direction of the internal iliac artery before deployment of the bifurcated graft section to further extend the side branch into the internal iliac artery.

The invention may form part of a delivery system for delivering a bifurcated prosthesis through a patient's vasculature, the bifurcated prosthesis having a main branch defining a first lumen and a side branch defining a second lumen, the delivery system comprising: a first guide wire; a delivery catheter having a lumen sized to accommodate the first guide wire, the delivery catheter being configured to be advanced through the patient's vasculature over the first guide wire; and a second guide wire configured to extend through the lumen of the side branch when the bifurcated prosthesis is loaded on the delivery catheter and being moveable with respect to the prosthesis.

### BRIEF DESCRIPTION OF THE DRAWINGS

FIG. 1 is a diagrammatic view of a portion of a human vascular system depicting an abdominal aortic aneurysm which extends from below the renal arteries and into a common iliac artery.
FIG. 2 is a view of the aneurysm of FIG. 1 with a main graft system in its deployed position, extending from just below the renal arteries to near the iliac bifurcation.
FIG. 3 is a side view of the bifurcated prosthesis that may be used with this invention.
FIG. 4 illustrates advancement of the delivery catheter of this invention along a main guide wire.
FIG. 5 illustrates advancement of the contracted bifurcated prosthesis into a leg of the main graft.
FIG. 6 illustrates the position of the retrograde core extending from the side arm of the bifurcated prosthesis in the internal iliac artery.
FIG. 7 illustrates the extension of the main branch of the bifurcated prosthesis into the internal iliac artery.
FIG. 8 illustrates deployment of the external branch of the bifurcated prosthesis in the external iliac artery.
FIG. 9 illustrates deployment of the side branch of the bifurcated prosthesis into the internal iliac artery.
FIG. 10 illustrates the fully deployed bifurcated prosthesis of FIG. 3 after the catheter has been withdrawn.
FIG. 11A is a top view and FIG. 11B is a side view of the bifurcated prosthesis with portions of graft material removed to show location of the stents.
FIG. 12A shows graft material from which the bifurcated prosthesis is fabricated and FIG. 12B shows the graft material after it has been sewn.
FIGS. 13A and 13C show end views of a support stent, and FIG. 13B shows a side view of a support stent, illustrating stitching on the main branch of the bifurcated prosthesis.
FIG. 14 shows an end view of a support stent illustrating stitching on the side branch of the bifurcated prosthesis.
FIG. 15 shows a perspective view of the arm stop located within the side branch of the bifurcated prosthesis.
FIG. 16 illustrates a partial cross section view of the end of side branch 34 showing the arm stop of FIG. 15.
FIG. 17 is a perspective view of the delivery system shown deploying the bifurcated prosthesis in FIGS. 4 to 9.
FIG. 18A is a cross section view of the handle located at the proximal portion of the delivery catheter of this invention and FIG. 18B is an enlarged view of a portion of the delivery catheter of FIG. 18A. FIG.18C is a view similar to FIG. 18A but with the delivery catheter rotated 90°
FIG. 19 is a cross section view of the distal portion of the delivery catheter of this invention.
FIG. 20 is a cross section along line a-a of FIG. 19.
FIG. 21A is a perspective view of the retrograde guide wire and FIG. 21B illustrates an exploded view of a portion of the retrograde guide wire of FIG. 21A.
FIG. 22 is a side view of a distal portion of the delivery system of this invention during deployment of the bifurcated prosthesis.
FIGS. 23A to 23F illustrate deployment of the main branch of the bifurcated prosthesis and relative motion of the monofilaments and suture ties.
FIGS. 24A and 24B are cross section views illustrating the sutures wrapped around the monofilament.
FIG. 25 is a cross section view illustrating the sutures within the planetary lumens.

### DETAILED DESCRIPTION OF THE PREFERRED EMBODIMENTS

The present invention is an apparatus to repair aortic aneurysms that extend into at least one common iliac artery and do not have a suitable region for seating the caudal end of a graft system by use of a stent or other attachment device. A bifurcated prosthesis is designed to be used in combination with a main graft system having legs extending into the common iliac arteries. The prosthesis provides the main graft system with a place to securely seat its iliac legs without blocking an internal iliac artery.

The terms "distal" and "proximal" as used herein refer only to the delivery catheters used to position and deploy the main graft system and the bifurcated prosthesis, not to the vasculature. The present method contemplates advancement of the delivery catheter in a retrograde manner (i. e., against the flow of blood). Therefore, "proximal" refers to a location closer to the physician and "distal" refers to a location farther from the physician. The vasculature is referred to with respect to the cranial (closer to head) and caudal (closer to feet) directions. Also, as used in this specification, the term "above", in the context of relative positioning, with respect to the aneurysm, refers to the regional cranial of the aneurysm, for example, within the aorta, whereas "below" refers to the region of the vasculature caudal of the aneurysm, for example, within the common iliac arteries.

Turning now to the drawings, the deployment of the bifurcated prosthesis into one of the legs of the main graft is shown.

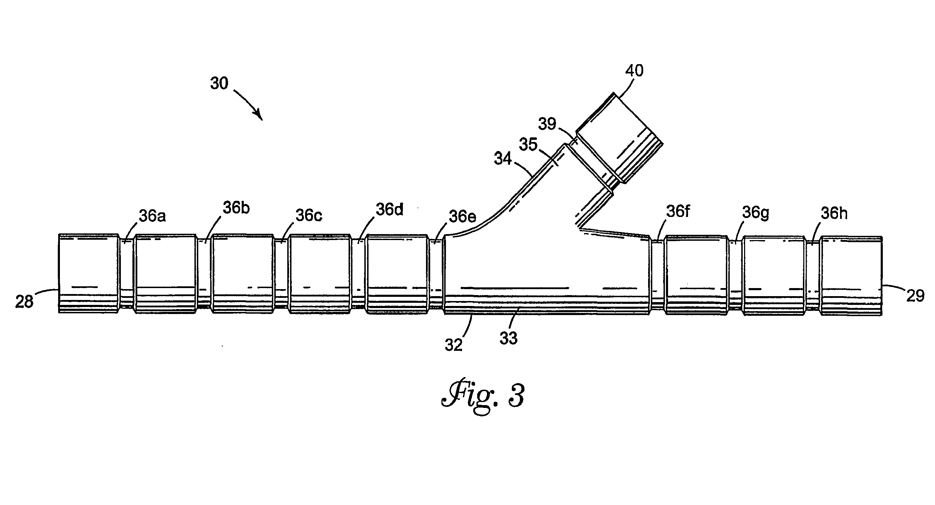

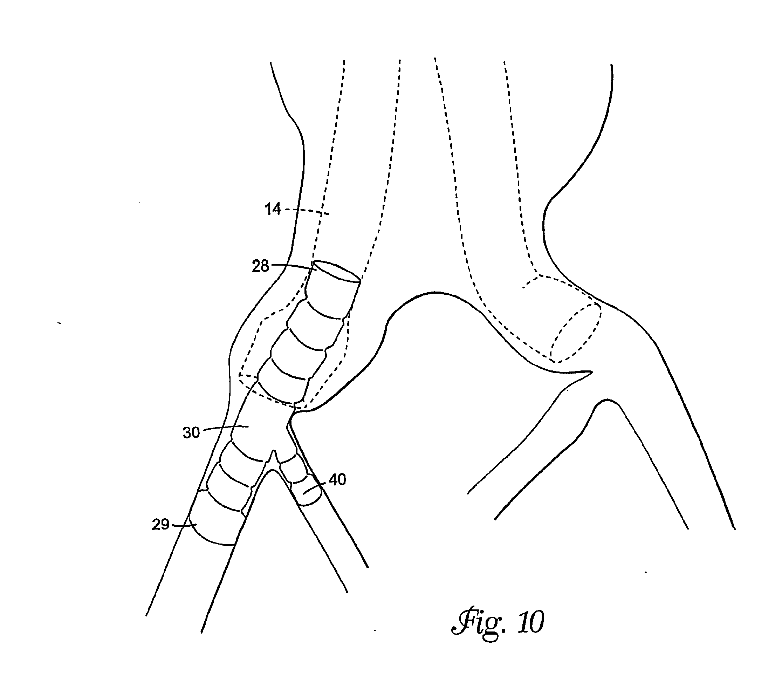

As best seen in FIG. 3, bifurcated prosthesis 30 (also referred to as the iliac bifurcation graft) comprises common/external iliac branch 32 (defining a main branch lumen) and an internal iliac branch 34 (defining a side branch lumen). The prosthesis includes graft tubing components 33 and 35, and a series of support stents (not shown). Circumferential crimps 36a to 36h are located along the main branch, as well as crimp 39 on the side branch. The support stents are preferably on the inside of the graft tubing, and are positioned within the regions of the graft tubing between the crimps, as well as at each of the three ends of the graft tubing (i.e., end 40 of side branch 34, and distal end 28 and proximal end 29 of main branch 32). The support stents are preferably thermally expandable nickel-titanium alloy. Both legs are generally tubular, having a circular cross-section. The common/external iliac branch has an upper portion which is positioned above the junction of the common iliac artery with the internal iliac artery and a lower portion which is positioned in the external iliac artery below the internal iliac artery. The internal iliac branch (side branch) is attached to and projects from the common/external iliac branch (main branch) and is positioned within the internal iliac artery. This can best be seen in FIG. 10. The attachment between the graft tubing and stents is preferably by sutures. The prosthesis is delivered by way of a delivery catheter in a first folded and contracted position. Once the prosthesis is positioned properly, the stents expand radially during deployment so that the branches of the bifurcated prosthesis are secured at the iliac bifurcation in their proper position.

The bifurcated prosthesis is advanced into the iliac artery by means of a delivery catheter. The prosthesis may be positioned at any time, however, in a preferred embodiment, the bifurcated prosthesis is placed following the deployment of a main graft system that extends from the aortic neck of the aneurysm into an iliac artery.

Delivery of the bifurcated prosthesis is herein described as following the placement of the main graft system such as the graft system described in the previously identified commonly assigned, co-pending U.S. application.

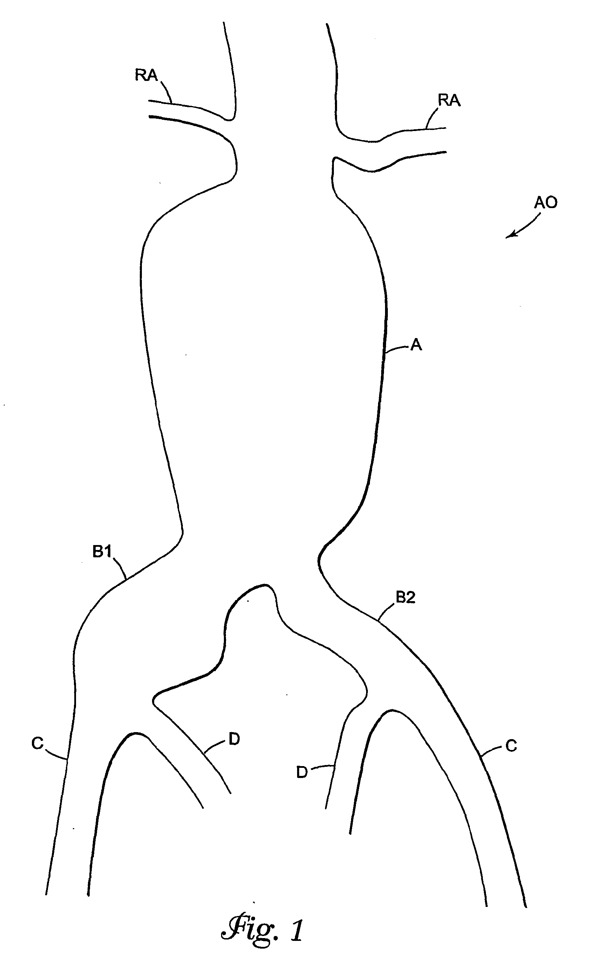

FIG. 1 illustrates aneurysm A in the infrarenal aorta Ao having a morphology that would benefit from use of the bifurcated prosthesis and delivery system of the present invention. The infrarenal aorta is that portion of the aorta disposed between the left and right renal arteries RA and the common iliac arteries B1 and B2 which branch right and left, respectively. Each common iliac artery branches into internal and external iliac arteries, D and C, respectively. External iliac artery C becomes the femoral artery below the inguinal ligament. Internal iliac artery D is also known as the hypogastric artery. As depicted in FIG. 1, right common iliac B1 is aneurysmal, and left common iliac B2 is not. This particular morphology will be utilized to illustrate the use of the graft and delivery system in this invention, but is in no way intended to limit the types of morphology in which this invention could be utilized.

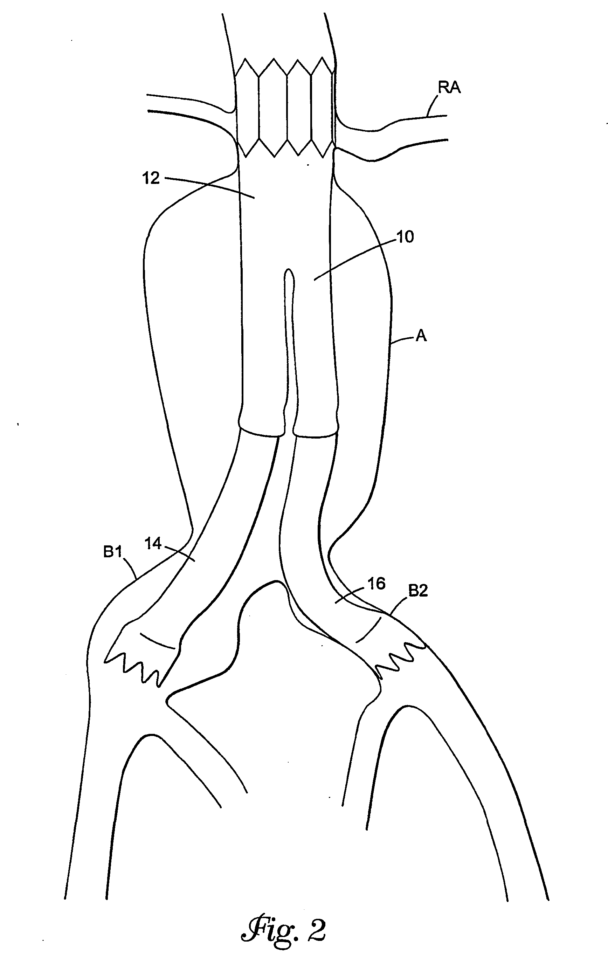

FIG. 2 shows main graft system 10 already deployed within the aorta and common iliac arteries. The main graft system comprises trunk portion 12, the cranial portion of which is attached to the vasculature just below the renal arteries, and legs 14 and 16 that extend toward right and left iliac arteries, respectively. The overlap of the main graft system with non-aneurysmal vessel at the upper end (just below the renal arteries) is suitable for a seal, as is the overlap in left common iliac B2. However, the morphology of the aneurysm does not provide a place to seat the lower end of the main graft system in the right common iliac. Thus the blood flow lumens created by the main graft system do not seal to the aneurysm. Therefore, this situation would benefit from placement of the bifurcated prosthesis graft of this invention.

FIG. 10 depicts the aortic anatomy in the region of the right iliac artery (B1) after placement of bifurcated prosthesis 30 into the right iliac arterial system. As shown in FIG. 2, there exists the possibility for leakage into the aneurysm around the lower end of right leg 14 of main graft system 10. In FIG. 10, the sealing between the blood flow lumens created by the main graft system and the aneurysm sac is formed at caudal ends 29 and 40 of the branches of bifurcated prosthesis 30. A seal is formed with the external iliac artery and the internal iliac artery. A seal between upper end 28 of bifurcated prosthesis 30 and main graft system 10 is also present.

In this manner, the complex morphology of this aneurysm is sealed robustly, and flow to both branches of the iliac artery are preserved.

### Delivery Steps

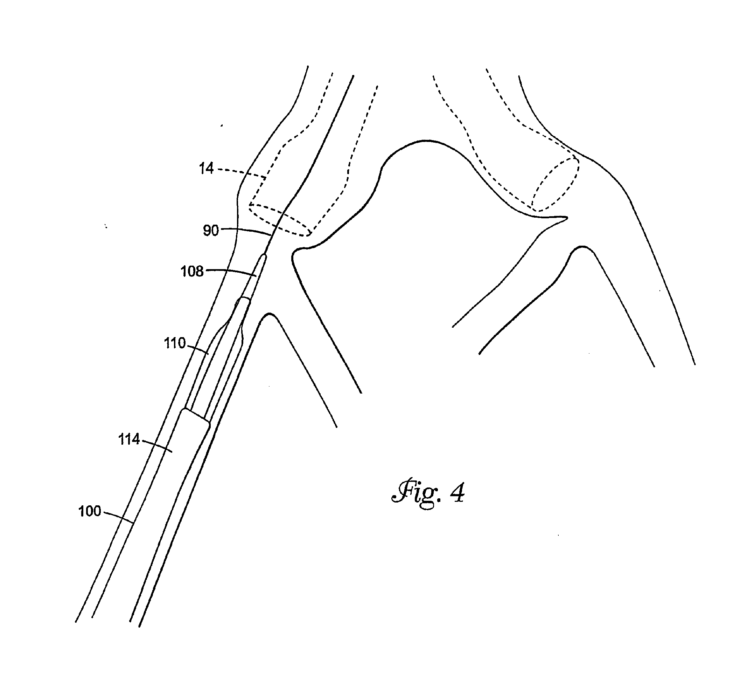

FIGS. 4 to 10 illustrate the delivery steps of the iliac bifurcation prosthesis into a main graft system that is in place and fully deployed. More specific details of the delivery catheter and bifurcated prosthesis are described below.

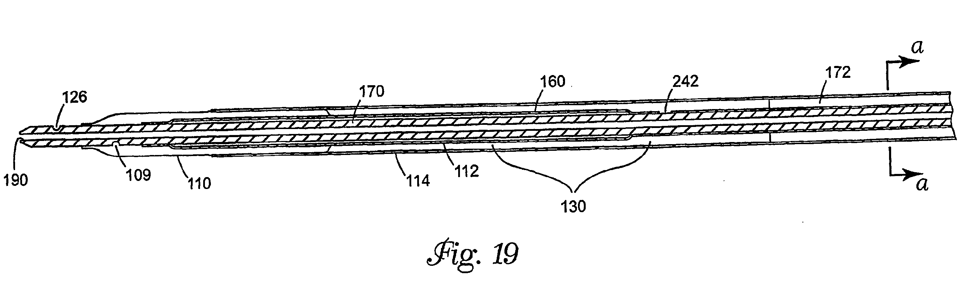

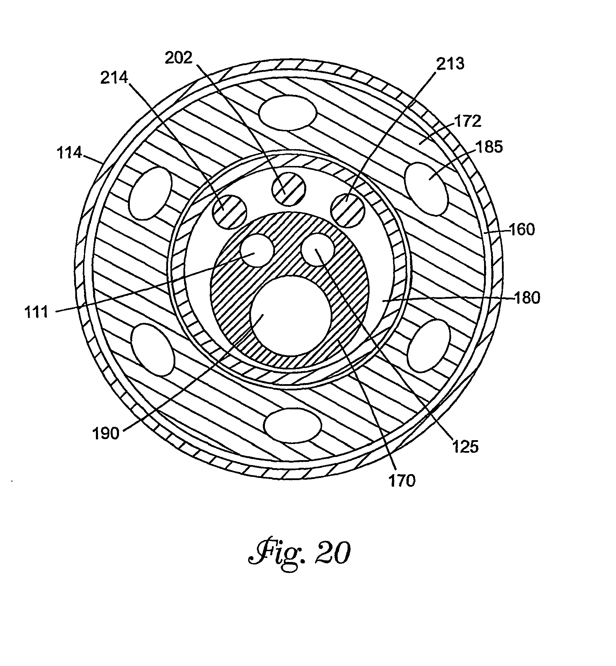

In FIG. 4, delivery system 100 for the bifurcated prosthesis is shown being advanced along a main guide wire 90 that had been previously put into place in order to deliver the main graft system. Delivery system 100 is advanced into leg 14 of main graft system 10 over main guide wire 90 which feeds through guide wire lumen 190 (such as shown in FIGS. 19 and 20). In this initial delivery state, distal tip 108 and inflated distal balloon 110 extend beyond the end of main sheath 114.

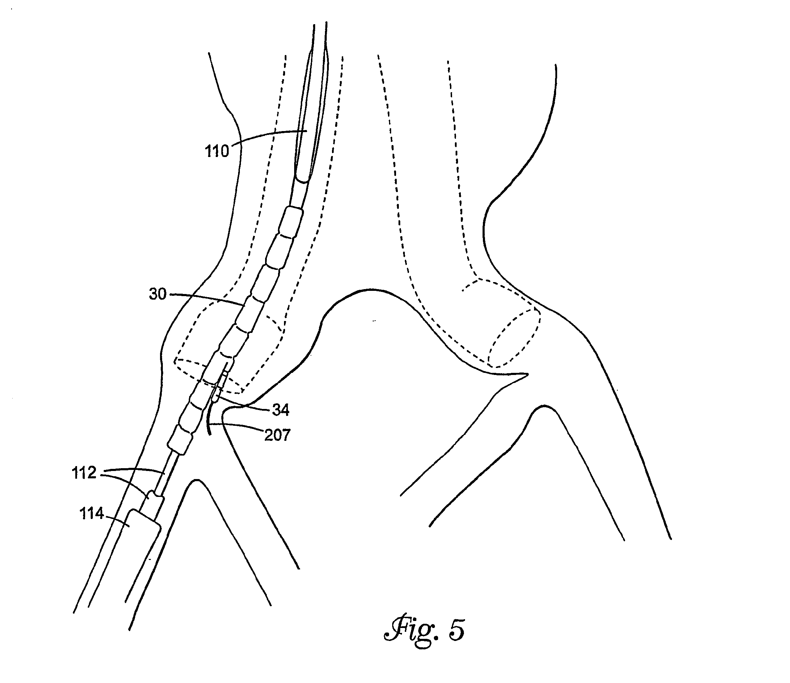

Delivery system 100 is positioned within the vasculature such that the side branch of the prosthesis is above the iliac bifurcation, but rotationally oriented in the plane of the iliac bifurcation. It is important that the side branch be properly rotationally oriented, otherwise the origin of the lumen of the side branch can be kinked off after delivery and expansion. Markers, described later, on the prosthesis as well as the platinum tip on the guide wire facilitate this orienting. As shown in FIG. 5, the balloon is deflated and the main sheath is retracted to expose the bifurcated prosthesis. At this point, the prosthesis is maintained in a contracted configuration by the use of chilled saline (as described in more detail below), together with a suture tie release mechanism, one on the main branch of the bifurcated prosthesis, and one on the side branch, to secure the graft material in a contracted position. Retrograde guide wire 200 has two portions: main core 202 and retrograde core 204, described in detail below. Tip 207 of retrograde guide wire 200 is shown protruding from side branch 34. This retrograde guide wire is used to facilitate advancement and tracking of the side branch into the internal iliac artery.

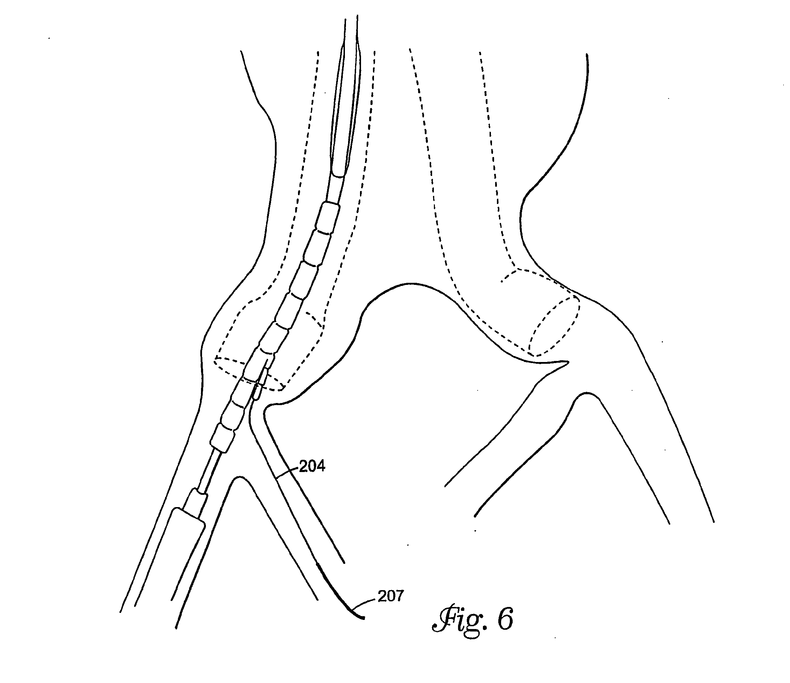

Retrograde core 204 is advanced further into the internal iliac artery, as shown in FIG. 6. Tip 207 of the core 204 is curved, as shown, and can be steered to facilitate navigation into and down the internal iliac artery. The advancement and steering is done from the handle of the delivery catheter, as described below.

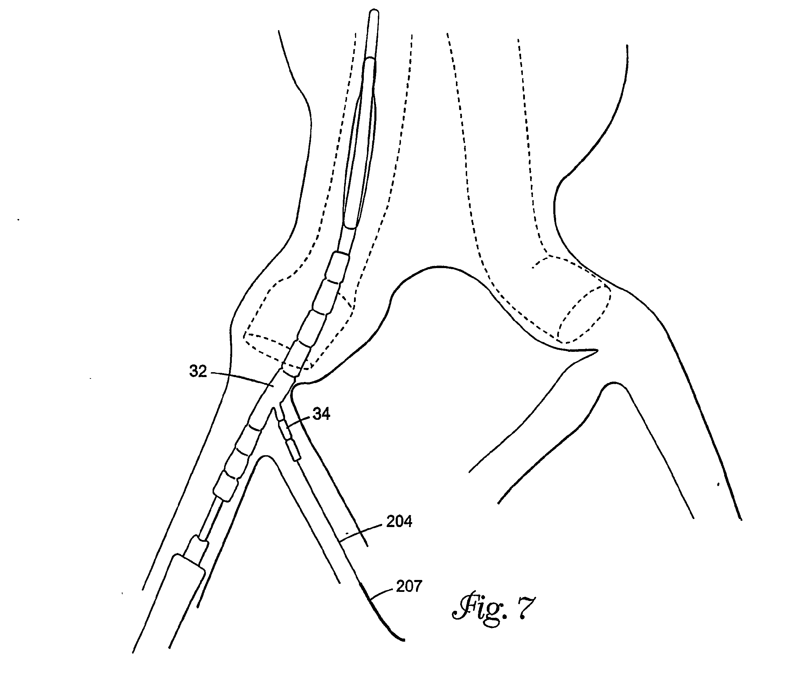

In FIG. 7, delivery system 100 is retracted in the proximal direction. As this is done, side branch 34 of bifurcated prosthesis 30 advances into the internal iliac vessel. The presence of the retrograde guide wire facilitates this side branch's tracking into the internal iliac. Depending on how the retrograde guide wire is manipulated at the handle, the retrograde wire will further advance together with the side branch, or its position can be maintained while the side branch is advanced into the internal iliac artery.

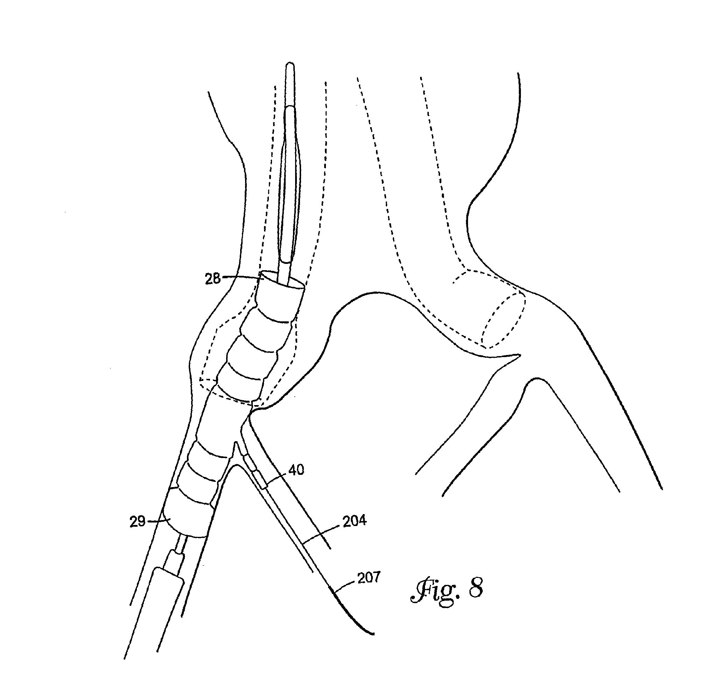

FIG. 8 illustrates the expansion of main branch 32 of bifurcated prosthesis 30. A tie mechanism secures the bifurcated prosthesis in a contracted condition when the bifurcated prosthesis is delivered and this is released and removed (described further below). The flow of chilled saline is also temporarily stopped, to allow for the stents in the main branch to warm to body temperature and expand the bifurcated prosthesis outward. Upper end 28 of main branch 32 is now expanded and in a sealing arrangement with the main graft leg 14, and lower end 29 of main branch 32 is expanded and in a sealing arrangement with the external iliac artery.

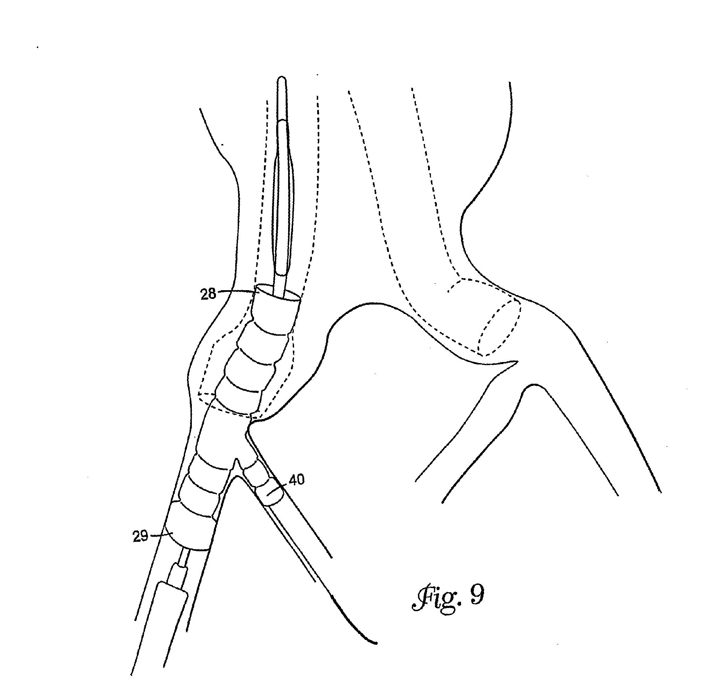

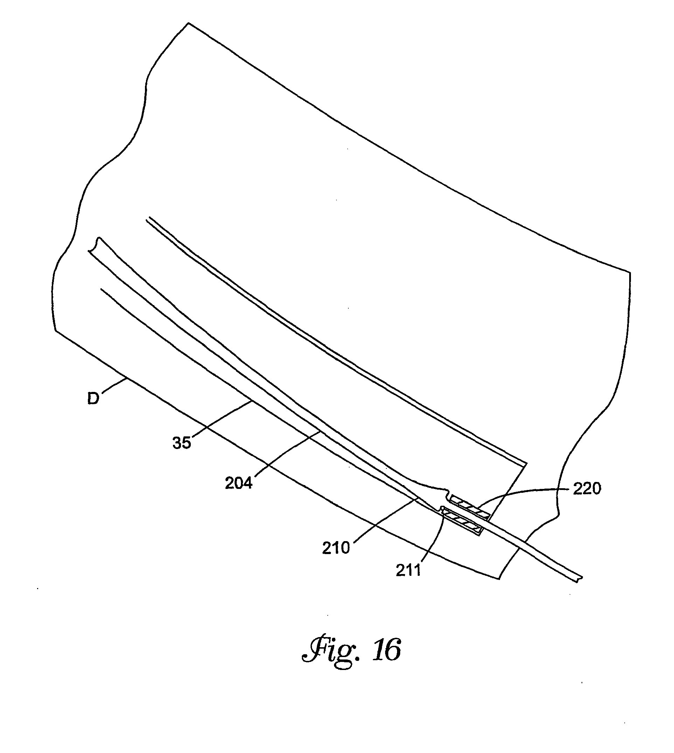

FIG. 9 illustrates the expansion of the side branch of the bifurcated prosthesis. The tie mechanism which secures the graft material of the side branch in a folded and compressed condition is released and removed. The flow of chilled saline is then stopped, again to allow for the stents in the side branch to warm up and thermally transform and expand. Once expanded, end portion 40 of side branch 34 is now expanded and in a sealing arrangement with the internal iliac artery, thus the vasculature on the right side (in this example) is effectively scaled from the aneurysm. The retrograde core is positioned toward the caudal side of the lumen of side branch 34. This is because the core traverses an arm stop that is attached on the inside of the bifurcated prosthesis, near the end of the side branch. This is described further below and shown in FIG. 16.

FIG. 10 illustrates a fully deployed bifurcated prosthesis after the removal of the delivery catheter. The retrograde wire is retracted fully inside the inner catheter, by advancing the main core in a distal direction. Then the delivery catheter is removed from the vasculature.

The delivery steps illustrate the use of a single bifurcated prosthesis at the right iliac bifurcation, however, it is contemplated that both left and right bifurcated prostheses could be delivered. Of course, a single bifurcated prosthesis could be delivered at the left iliac bifurcation using the same steps as described for delivery to the right iliac bifurcation. It is also contemplated that the bifurcated prosthesis could be used in other parts of the anatomy involving bifurcating conduits.

### Bifurcated Prosthesis Assembly

The bifurcated prosthesis is illustrated in FIGS. 3, and 11 to 14. The construction, materials, and properties of the prosthesis and support stents are similar to those described for the endovascular graft system disclosed in commonly assigned, co-pending application referenced above.

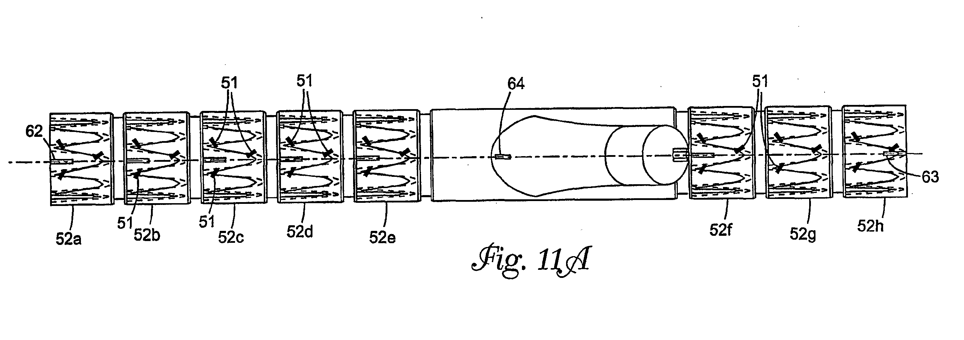

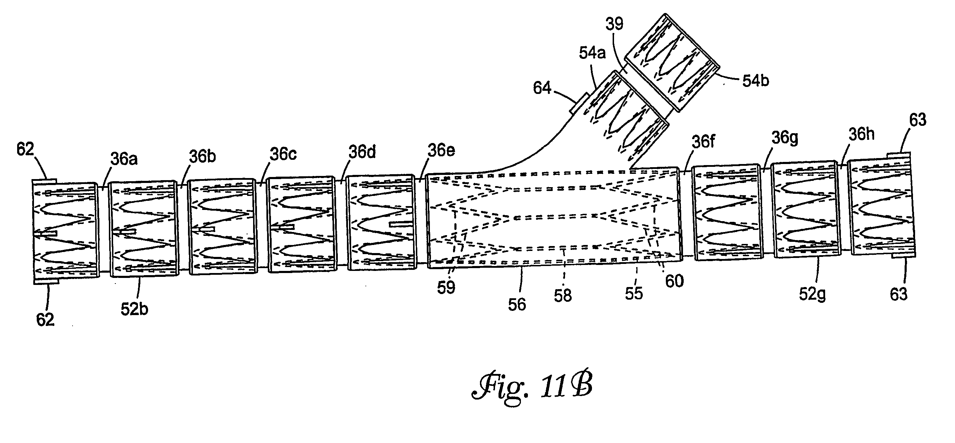

FIGS. 11A and 11B are views of the bifurcated prosthesis with portions of the graft material removed in order to show the individual support stents on the inside of the graft material. FIG. 3 shows a side view of the prosthesis, showing the tubular graft material. Support stents 52a to 52h are attached to the internal surface of the graft material in main branch 32 which defines a first graft conduit and support stents 54a and 54b are in side branch 34 which defines a second graft conduit. The tubular graft material has a series of heat set circumferential rings or crimps 36a to 36h on main branch 32 and crimp 39 on side branch 34. The crimps define circumferential "pockets" inside which are the individual support stents. Cage stent 55 is provided at the junction of the side branch and the main branch.

The support stents and the cage stent comprise nickel titanium alloy, and are formed by laser cutting of tubing. The processing steps including shape setting, grit blasting, and electropolishing. The support stents are formed into a zigzag pattern.

Cage stent 55 comprises regions 56 and 57 of circumferential zigzags formed of connecting portions 59 and 60 on each end, and longitudinal struts 58 that connect zigzags 56 and 57. The longitudinal struts are not evenly spaced around the periphery of the cage stent; that is, there is more open area between two of the struts than between remaining struts. This can be accomplished by forming a cage stent with evenly spaced struts and then removing one strut before placing it in the bifurcated prosthesis. This region of additional space is placed adjacent to the side branch. This open area in the stent assists in preventing disruption in the flow of blood into the side branch.

It is contemplated that the diameters of various portions of the bifurcated prosthesis can be tailored to a variety of dimensions, depending on the dimensions of the anatomy, as well as the dimensions of the main graft, if previously deployed in the aorta and aortic bifurcation. For example, the expanded unrestrained diameters of each end of the main branch, and for the side branch can be tailored such that the diameter of the first end of the main branch is D1, the diameter of the second end of the main branch is D2 and the diameter of the side branch is D3. The prosthesis can be tailored such that D1, D2 and D3 are not equal. Ordinarily, D3 will be less than either D1 or D2. Further, the length of the main branch and side branch can vary as well as the number of stents contained within each branch. For illustration purposes, dimensions suitable for deployment into a main graft system having a leg diameter of about 10-11 mm (above the bell bottom shown in FIG. 11), an internal iliac lumen of about 7-8 mm, and an external iliac lumen of about 6-10 mm are described.

In this anatomy, the preferred unrestrained expanded diameter of the main branch is 11 mm (in the uncrimped regions, the crimps have a reduced diameter of approximately 10 mm) throughout its length. The unrestrained expanded side branch is preferably 9 mm in diameter in the uncrimped regions, and 8 mm in diameter in the crimped regions. The individual stents on the inside preferably have unrestrained expanded diameters approximately equal to their respective branch diameters. These dimensions are suitable for the deployed bifurcated prosthesis to form a seal for a range of vessel lumen diameters. Internal iliac lumens ranging from about 7 to 8 mm, and external iliac lumens ranging from about 6 to 10 mm in diameter could be treated with the prosthesis of the above diameters.

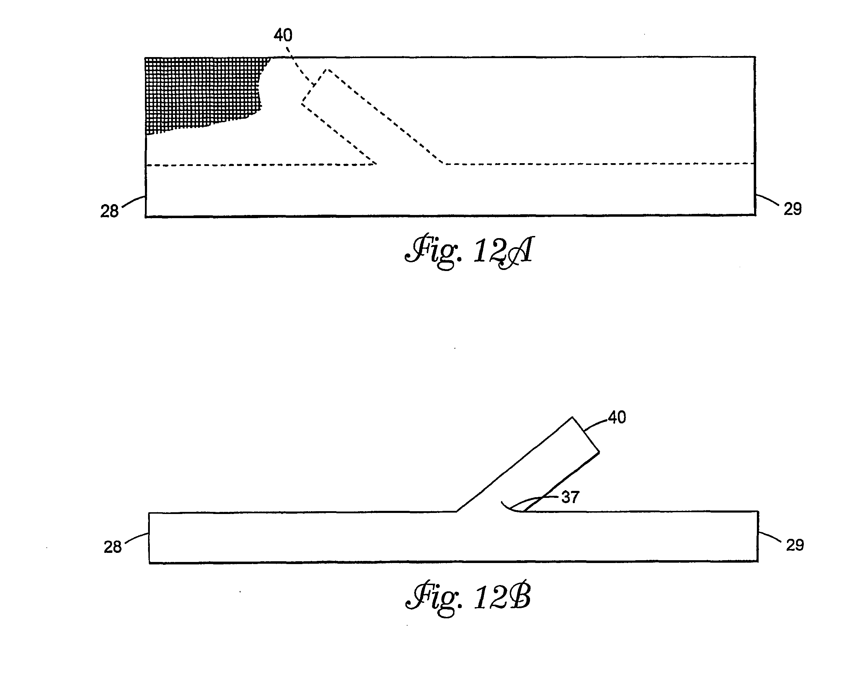

As illustrated in FIG. 12B, a number of individual stents is utilized in each branch of the bifurcated prosthesis. While the total number of individual stents could vary, in a preferred embodiment, there are 5 individual zigzag stents above the cage stent, and 3 below. The side branch preferably contains 2 individual stents. The total length of the main branch in a preferred embodiment is about 11 cm, and the total length of the side branch is about 2 cm (measured along the caudal edge).

As shown in FIG. 12A, formation of the graft material into the shape necessary for the bifurcated prosthesis begins with an initial woven graft tube. Alternatively, a sheet of graft material can be folded so that the stitching described below is done above the fold line where the material consists of two layers. The preferred material for the graft tube is a woven polyester yarn, and more preferably a yarn of 40 denier, 27 filament polyester, such as that available under the commercial designation "Dacron". The graft tube is preferably 250 ends/inch (about 98 ends/cm) and 125 picks/inch (about 49 picks/cm). When flattened, the tube is sufficiently wide for the bifurcated prosthesis. The tube is sewn, preferably using a straight stitch of about 0.5 mm spacing, with a bobbin thread. The stitching is indicated in FIG. 12A by the dotted line. The stitching threads are preferably 5-0 polyester suture. The portion of the tubular graft material which becomes the side branch is oriented in a reverse angle (to that of the finished angle), so that end 40 of side branch graft material 35 is closer to end 28 of main branch graft material 33. After the stitching is completed, any excess material from the graft tube is trimmed away close to the stitch line, preferably about 1.0 mm away. The trimming can be done with a hot soldering iron, which fuses loose filaments of the yarn. The graft material is then turned inside out so that the seams are on the inside. Now the side branch is rotated such that end 40 faces lower end 29 of the main branch. As a result, fold 37 is formed at the base of the side branch. The tubular graft material is then placed on the outside of a solid mandrel, which has the shape of the tubular graft material, together with the crimps. The tubular graft material is heat set on this mandrel, forming the crimps, pressing out the seam (forming a "butterfly" seam), and tightly creasing fold 37 at the base of the side branch.

Fold 37 allows for the angle of the side branch 34 of bifurcated prosthesis 30 to be varied without kinking. This is important because the anatomy of the iliac bifurcation varies from patient to patient. The fold accommodates a wide range of angles for the side branch, while the lumen of the side branch remains open. This controlled, creased fold provides a way to control the angle of the side branch. This also helps avoid any problems created by wrinkles that might otherwise form and that could protrude into the lumen of the side branch, resulting in disrupted blood flow.

As shown in FIGS. 11A, 13 and 14, the individual support stents are placed within the "pockets" and attached to the graft material with point stitches 51. Zigzag support stents 52a to 52h, 52a, 52b are secured at some of their apices with point stitches 51. Although not shown, the zigzag ends of cage stent 55 are similarly secured. In order for the bifurcated prosthesis to be folded and compressed in the preferred manner, only particular apices are secured to the graft material. There are point stitches on the top-most three apices of each zigzag, as well as the bottom most three apices. This leaves the sides of the stents and graft material free from direct securement via point stitches.

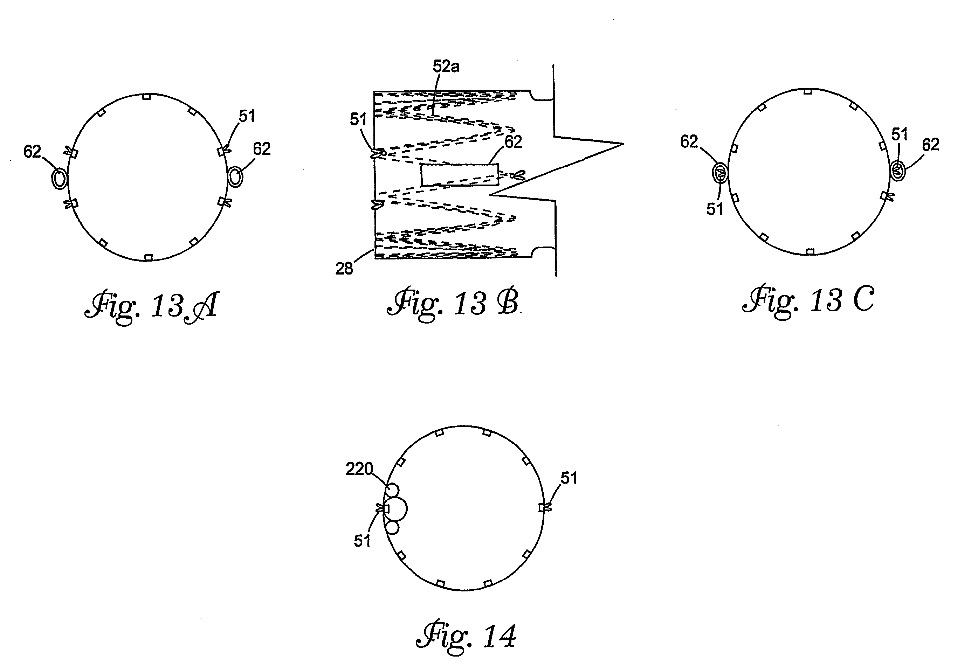

FIGS. 13A to 13C show end and side views of the stent used at end main branch 32. Point stitches 51 can be seen on either side of radiographic marker 62 in FIG. 13A. A side view of marker 62 is shown in FIG. 13B. Point stitches 51 can seen in FIG. 13C at the stent end 29 opposite the marker. Similarly, FIGS. 14A to 14C show end and side views of the distal end of the stent used at the end 40 of side branch 34. Point stitches 51 can be seen in the end views of FIGS. 14A and 14C.

Each support stent throughout the bifurcated prosthesis has six point stitches 51, and cage stent 55 has eight points stitches 51 at four ends each on the zigzag portions. The point stitches are preferably formed from polyester braided 5/0 surgical suture impregnated with PTFE (polytetrafluoroethylene) strands. A preferred suture material is Silky II Polydeck™ by Genzyme.

Each stitch is a 4-layer square knot surrounding an apex of the stent. Preferably, the first two layers have a double wrapping. The free ends of the knotted suture material are then trimmed close to the knot, and further melted back to prevent the unraveling of filaments in the suture material. To allow the ends of the bifurcated prosthesis to be contracted in the lowest possible profile for delivery, the square knots that reside at all three ends of the bifurcated prosthesis are 2-layer square knots. This minimizes the chance that the sutures will bang up on the end knots.

A set of radiopaque markers is attached to the bifurcated prosthesis, to aid in the positioning of the device during delivery. At both ends of main branch 32 are sets of tubular markers 62 and 63 as shown in FIG. 11B. The tubular markers are secured to the graft material with sutures, as shown in FIGS. 11B, 13A and 13B. The markers preferably are fabricated of platinum alloy, and are 0.100 inches long (0.254 cm), and 0.015 by 0.023 inches (0.038 by 0.058 cm) diameter, preferably. They are attached in a plane defined by the central axis of the main branch and the side branch. In this position, the rotational orientation of the contracted bifurcated prosthesis can be determined, so as to properly orient the side branch with the internal iliac artery. To minimize the added bulkiness of the markers, they are attached to the graft material between struts, and they are further flattened radially. Another marker 64 is positioned on the cranial side of the origin of the side branch.

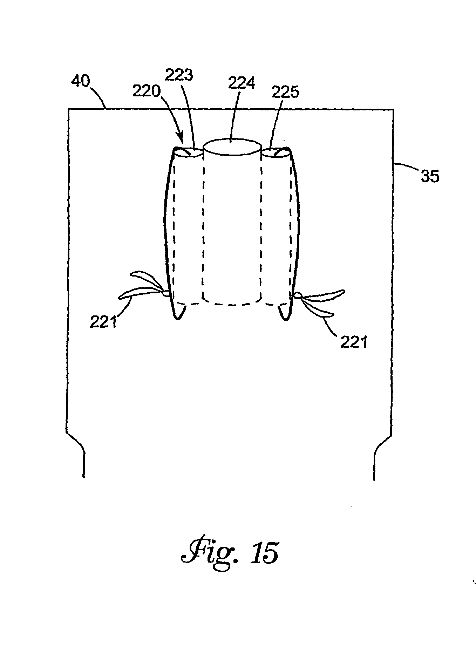

FIGS. 15 and 16 illustrate the arm stop and how it engages the retrograde core 204. FIG. 15 is a partial view of the lumen of side branch 32. Near end 40 of side branch 34, arm stop 220 is attached to the inside surface of graft material 35 of side branch 34 by means of sutures 221. FIG. 16 illustrates a partial cross section view of the end view of side branch 34. Arm stop 220 is attached on the caudal end of the graft material.

The arm stop comprises three small tubes 223, 224, and 225. Central tube 224 receives retrograde core 204. Two smaller tubes 223 and 225 adjacent the central tube are attached by welding, and are used to attach arm stop 220 to the graft material via sutures. The material of the tubes comprising the arm stop are preferably the same material as that for the stents, a nickel titanium alloy. It is preferable to use the same material because the arm stop does come in direct contact with the stent near the end of the side arm. To minimize the chance of galvanic corrosion, it is preferred to use the same alloy for metal structures which contact each other. The arm stop is secured toward the caudal side of the opening at the end of the side branch, as indicated in FIGS. 9 and 16. By locating the arm stop on this side of the opening, the side branch, when still contracted, as depicted in FIGS. 6 and 7, can be more easily tracked into the iliac bifurcation. Locating the arm stop here minimizes the chance for the caudal edge of the end of the side branch getting hung up or snagged on the top of the iliac bifurcation.

The purpose of the arm stop is to allow for the portion of the retrograde core 204 that traverses the side branch to engage with the end of the side branch. Retrograde core 204 is initially located within central tube 224 of arm stop 220. There is an increased diameter region, or bump 210 having shoulder 211 on retrograde core 204 that butts up against the cranial edge of arm stop 220 during the wire advancement step shown in FIGS. 6 and 7. Particularly after the step shown in FIG. 8, if the retrograde guide wire is firmly pushed out the side branch, it will place the entire side branch into tension, which helps facilitate full advancement of the side branch into the internal iliac artery. Without this mechanism to place or maintain tension on the side branch of the bifurcated prosthesis, it could have a tendency to bunch up as it is advanced into the internal iliac artery. This could result in incomplete placement into that artery as well as suboptimal deployment.

FIG. 16 illustrates the interaction of the arm stop 220 and the retrograde core 204. Further details on the preferred construction of the guide wire are provided below. Retrograde core 204 is shown within the contracted side branch 34 of bifurcated prosthesis 30 at a representative position in FIG. 8. Shoulder 211 of retrograde core 204 serves to engage distal end 40 of side branch 34, placing it under tension when the retrograde core is advanced or urged distally. The ability to periodically apply tension to side branch 34 is important to assure that side branch 34 is fully advanced into the internal iliac artery D prior to its expansion. This tensioning takes out any axial compression or "bunching" in the side branch 34.

There are a number of times during the delivery of bifurcated prosthesis 30 when retrograde core 204 is manipulated to apply tension to the side branch 34. The step shown in FIG. 6 includes the advancement of the retrograde core 204 into the internal iliac artery D, and also preferably includes a tensioning of the side branch 34 once retrograde core 204 engages with the arm stop 220. After the step depicted in FIG. 7, the primary advancement of side branch 34 into the internal iliac D, side branch 34 preferably is again tensioned. And finally, after the step shown in FIG. 8, the deployment of main branch 32, it is desirable to re-tension side branch 34, assuring it is as fully advanced into the internal iliac artery D as possible. If side branch 34 is not re-tensioned after this step, the expansion of the main branch 32 can push on the proximal end of the side branch and cause it to bunch or axially compress. Deployment of the side branch 34 with this compression can result in excess folds and kinks into the lumen formed by side branch 34.

### Delivery System

In the practice of this invention, the bifurcated prosthesis is delivered after a main graft system (such as 10 shown in FIG. 2) has been delivered into the vasculature and deployed by two delivery catheters. A first catheter delivers the aortic trunk and a first leg, and a second catheter delivers the second leg graft into the second branch of the aortic trunk, and is delivered from the opposite femoral artery as the first delivery system. Such a procedure is described in, for example, the commonly assigned, co-pending U.S. patent application referenced above. The bifurcated prosthesis of this invention is then delivered and deployed by a delivery catheter as herein described and shown in the Figures.

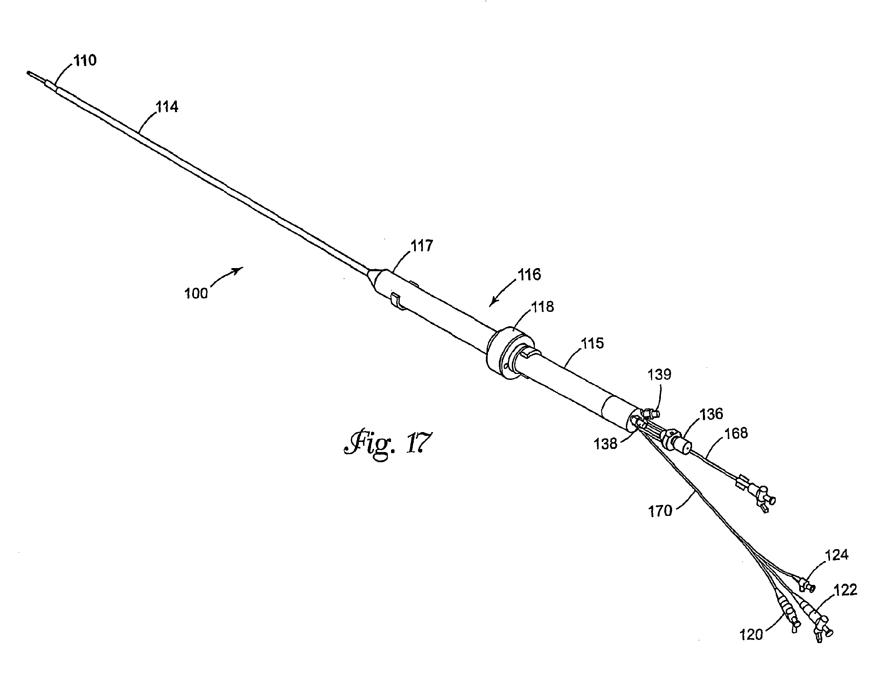

FIGS. 17 to 20 illustrate the delivery system of this invention. FIG. 17 is a perspective view of delivery system 100 including outer sheath 114 and handle 116. Outer sheath 114 retracts to expose inner catheter 112. Inner catheter 112 includes those portions of the catheter contained within outer sheath 114. Outer sheath 114 and inner catheter 112 are connected to handle 116. Outer sheath 114 retracts to expose inner catheter 112. Handle 116 at the proximal end of the delivery system causes relative sliding motion between sheath 114 and inner catheter 112 to facilitate delivery of bifurcated prosthesis 30. With reference to FIG. 19 the bifurcated prosthesis (not shown) is positioned in a contracted state in annular space 130 between outer sheath 114 and inner catheter 112. Although the delivery catheter is described herein as including an outer sheath it is within the scope of the present invention to deliver the bifurcated prosthesis with a delivery catheter which does not include an outer sheath. This is possible because the bifurcated prosthesis includes the suture tie down feature discussed hereafter.

The delivery catheter contains three fluid delivery or access ports having connector/adaptors 120, 122, and 124 as shown in FIG. 17. These connectors branch from the proximal end of inner shaft 170. First port/adaptor 120 communicates with balloon structure 110 near the distal end of the inner catheter, second port/adaptor 122 communicates with the main guide wire lumen 190, over which the delivery catheter is positioned, and third port/adaptor 124 communicates with an outlet 125 on the inner catheter for delivery of radiopaque contrast media. These fluid delivery and access lumens will be discussed in more detail below. An additional fluid port 168 allows for the infusion of chilled saline into the delivery catheter and around the contracted prosthesis, prior to its deployment.

The delivery system includes sheath 114, handle mechanism 116, inner catheter 112, retrograde guide wire 200, and three fluid delivery ports. Handle mechanism 116 has distal housing 117 and proximal housing 115, with sheath retraction knob 118 in between. Main guide wire lumen 190 extends through inner catheter 112 from the distal tip of the delivery system to the proximal end. This main guide wire lumen is adapted for use with typical 0.035 inch (0.089 cm) guide wires, such as those used for various endovascular procedures.

Outer sheath 114 of the delivery system is comprised of a two layer polymeric tube. The inner layer is preferably polytetrafluoroethylene (PTFE), and the outer layer is preferably polypropylene, which is treated to heat shrink it to the outside of the PTFE tubing. This results in a sheath which is lubricious on both the inside and outside, and has relatively high tensile stiffness, so as not to stretch significantly when the sheath is withdrawn off of the prosthesis. In a preferred embodiment, this sheath is approximately 0.250 inch (0.635 cm) ID, and 0.282 inch (0.716 cm) OD, for delivery of the bifurcated prosthesis of the dimensions described below.

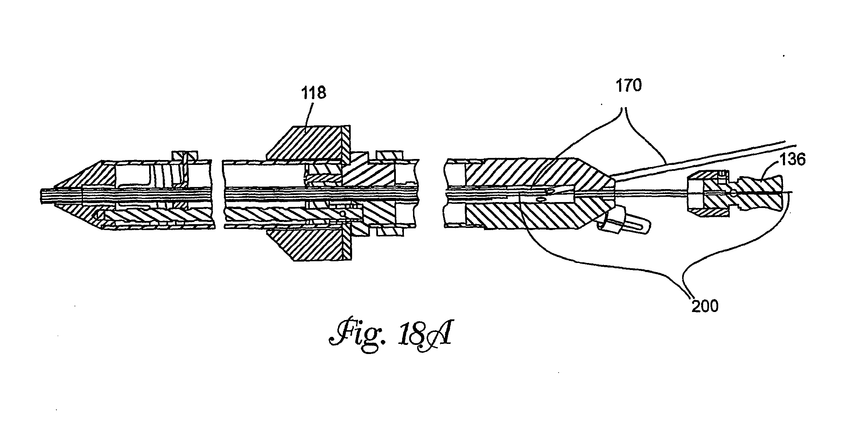

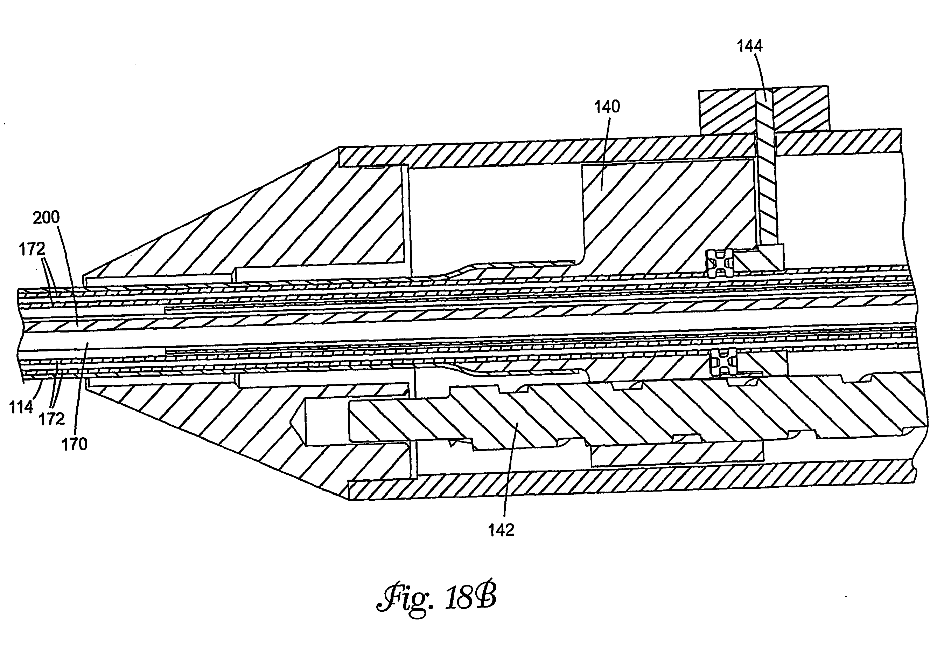

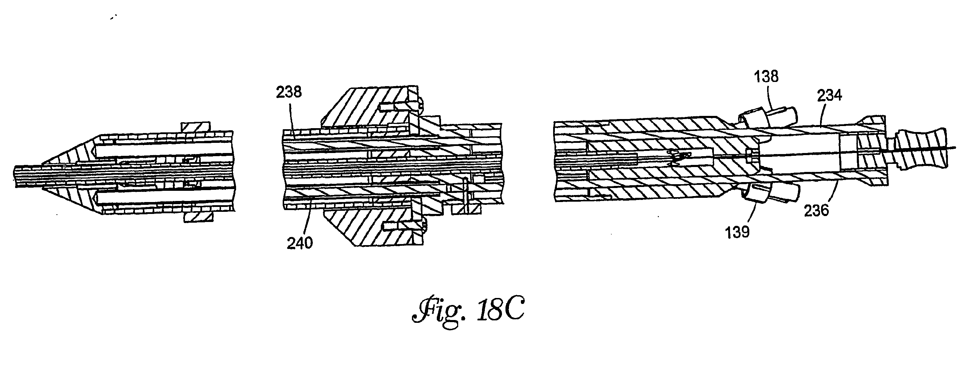

FIG. 18 is a partial longitudinal section view of the handle of the delivery catheter. FIG. 18B is an enlarged view of a portion of the distal region of the handle. FIG. 18C is a view similar to FIG. 18A but rotated 90°. The proximal end of the sheath is mounted on sheath mount 140, as shown in FIGS. 18A,18B and 18C, inside distal housing 117 of handle mechanism 116. In FIGS. 18A and 18B, the handle is shown in longitudinal section, except for the inner shaft 170 and the main core 202 of retrograde guide wire 200. Sheath retraction knob 118, when rotated, rotates threaded screw 142, which in turn moves sheath mount 140 in a proximal direction. The sheath mount and the sheath are initially in their forward or distal most position during the introduction of the catheter into the patient. (In FIG. 18A, the sheath mount is shown in its fully forward position). Sheath movement pin 144 is secured to distal housing 117 to prevent inadvertent movement of the sheath. Pin 144 engages with passageway 141 in the sheath mount and passageway 119 in distal housing 117, thus locking sheath mount 140. Once the sheath is desired to be withdrawn, as indicated in FIG. 5, sheath movement pin 144 is removed, and retraction knob 118 rotated. Sheath 114 and sheath mount 140 move longitudinally, relative to handle mechanism 116 , and relative to inner catheter 112 and contracted prosthesis 30. The inner catheter is in a fixed position relative to handle mechanism 116 and contracted prosthesis 30.

FIG. 19 depicts the distal region of the delivery system. For purposes of clarity, the contracted prosthesis, which is initially loaded into the delivery catheter, is not shown. It occupies a space between the sheath and the inner catheter indicated at 130. Retrograde guide wire 200 is also positioned within the inner catheter but is not shown in FIG. 19.

FIG. 19 shows distal balloon 110, saline delivery extension tube 160, inner shaft 170, and proximal shaft tube 172. Inner shaft 170, as described earlier, provides lumen 190 for main guide wire 90. As best seen in FIG. 20 this shaft also contains two other lumens, 111 for inflation and deflation of the distal balloon, and 125 for delivery of contrast media. Inner shaft 170 preferably comprises a single extrusion of tubing with three lumens, preferably formed of a thermoplastic polymer, such as PEBAX™ 7033. Exit port 126 for contrast lumen 125 is indicated near the distal tip of the inner shaft, and exit port 109 for inflation/deflation lumen 111 is shown near the distal end of the balloon. The inner shaft extends through the full length of the delivery system, and emerges out the proximal end of the proximal housing, to which it is adhesively bonded. At the proximal end of the inner shaft, each of the lumens is separated into a series of manifold/luer adapters, as shown in FIG. 17. Connector/adaptor 122 receives the main guide wire, 120 connects to the balloon inflation lumen, and 124 connects to the contrast delivery lumen.

Distal balloon 110 is attached at its distal end to the outside of inner shaft 170, as shown in FIG. 19, and at its proximal end to the outside of saline delivery extension tube 160. The inflated balloon is preferably slightly larger in diameter than the inner diameter of the sheath. In this manner, the distal balloon will sealingly engage the sheath and form a gradual diameter transition from the tip of the inner shaft to the sheath. The inflated balloon also provides for a smooth transition in stiffness, allowing the relatively stiff delivery system to track in tortuous vasculature. Saline delivery extension tube 160 is bonded at its distal end to the outside of inner shaft 170, and the proximal end is bonded to the central lumen of proximal shaft tube 172 in a region inside the balloon 110. It is the outside surface of the proximal end of the saline delivery extension tube that is bonded to the inside surface of the distal end of the proximal shaft tube. These are adhesively bonded together. Proximal shaft tube 172 then extends proximally within the sheath, and to the proximal end of the handle housing, where it is bonded in place. As shown in FIG. 17, fluid port 168 emerges which communicates with the central lumen formed by proximal shaft tube 172. During the delivery of the prosthesis, chilled saline is introduced into the delivery catheter via this central lumen.

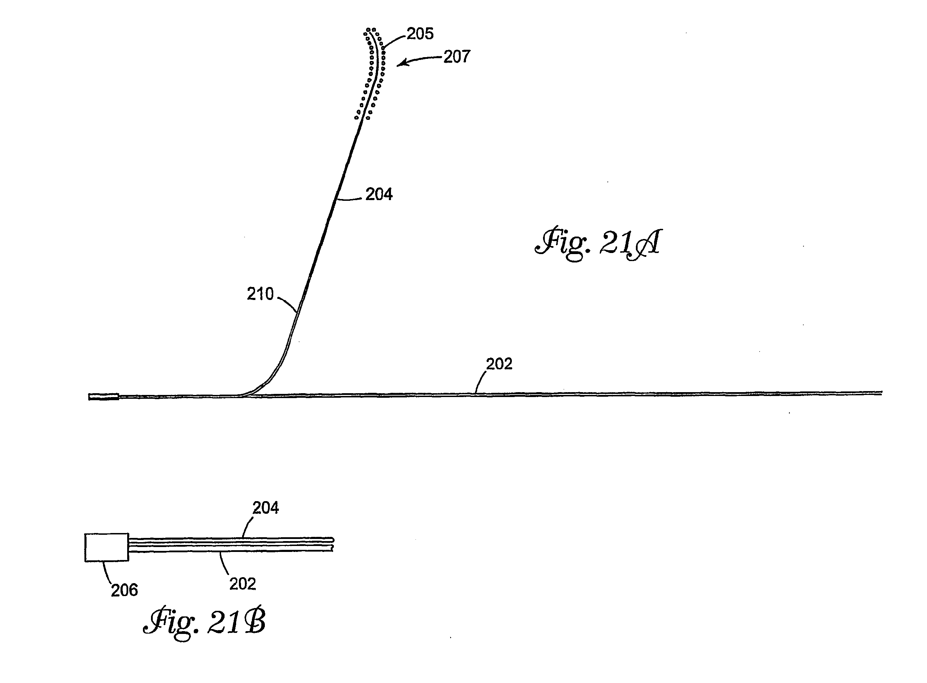

Saline delivery extension tube 160 has an enlarged diameter region towards its distal end. The space between this enlarged diameter and the inner shaft is where retrograde guide wire 200 resides. As best seen in FIGS. 21A and 21B retrograde guide wire 200 has two portions, main core 202 and retrograde core 204. The distal most portion of the main core is attached side-by-side with the proximal end of the retrograde core, as shown in FIG. 21A, via a swaged and soldered connector tube 206. Core 204 also is provided with coil tip 205 which provides support to core end 207. Coil tip 205 (shown with an exaggerated diameter) also assists in the radiographic imaging of the core end. The connector tube resides in the enlarged diameter region of the saline delivery extension tube 160 as shown in FIG. 19. When loaded into the delivery catheter, the curved regions of the retrograde guide wire are made to rest in a straightened configuration, but as depicted in FIGS. 6 and 7, once the sheath is withdrawn, the curved portions of the retrograde core help to angle the side branch away from the main branch, so as to facilitate tracking down the internal iliac artery. The main core of the retrograde guide wire extends from the connector proximally through the central lumen of the proximal shaft tube 172, and emerges out the proximal end of the handle mechanism terminating at the retrograde guide wire control mechanism, such as shown in FIGS. 17, 18A and 18C.

Steering knob 136 shown in FIGS. 17, 18A and 18B allows for rotation of main core 202 of the retrograde guide wire. This rotation causes the connector and retrograde core 204 and coil tip 205 to rotate as well (as shown in FIG. 21A), imparting steerability to the wire. As the knob is rotated, main core 202 and retrograde core 204 twist about each other. Therefore the amount of rotation in either direction is somewhat limited. The steering knob is mounted to advancement sliders 234 and 236 which are slidably received by alignment tubes 238 and 240. This allows the retrograde guide wire to be advanced and withdrawn into the handle mechanism, causing longitudinal movement of the retrograde core in and out of the side branch of the bifurcated prosthesis. Advancement of steering knob 136 into handle 116 causes retraction of retrograde core 204 into side branch 34, whereas withdrawal of steering knob 136 causes advancement of retrograde core 204 from side branch 34 and into the internal iliac artery. The alignment tubes extend through the sheath mount which prevents the sheath mount from rotating during sheath withdrawal.

Main core 202 of retrograde guide wire 200 preferably is comprised of 304 stainless steel wire of about 0.032 inches (0.081 cm) diameter for most of its length. The distal portion (about 10 inches (about 25.4 cm)) is centerless ground to about 0.020 inches (0.051 cm) diameter. The retrograde core is preferably fabricated of nickel titanium wire, and heat set in the shape shown in FIG. 21A. The proximal portion of this core is preferably approximately 0.018 inches (0.045 cm) diameter, with a "bump-up" in diameter to 0.026 inches (0.066 cm). At the distal end of this bump is an abrupt shoulder, and the diameter changes back to 0.018 inches (0.045 cm). The function of this bump has been previously described, as it engages with the smaller diameter of the arm stop, to allow for tension to be placed on the side arm during advancement of the side branch into the internal iliac artery. Further distally on the retrograde core, the diameter is gradually tapered to 0.0022 inches (0.0056 cm) at the distal-most end. The distal 2 inches (5.1 cm) of the core are covered with a radiopaque platinum alloy coil, with a coil diameter of preferably 0.017 inches (0.043 cm).

Referring to FIG. 19, when retrograde guide wire 200 (not shown in FIG. 19) is positioned in the delivery catheter, most if it resides within the inner catheter. However, tip 207 of retrograde core 204 emerges from the wall of the saline delivery extension tube at port 240, close to the contracted side branch of the bifurcated prosthesis 30. In this manner, once sheath 114 is withdrawn, as shown in FIG. 5, the tip of the retrograde core is exposed.

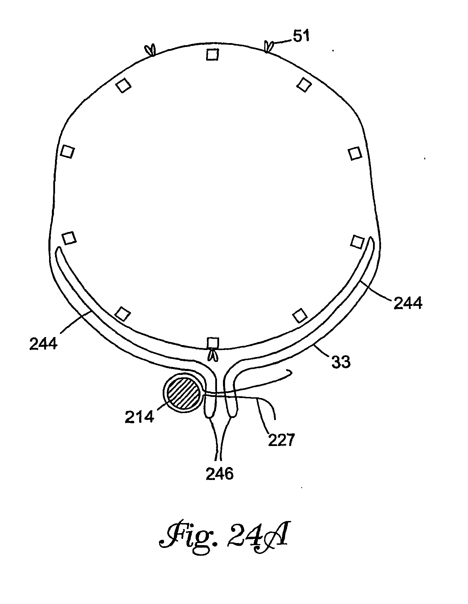

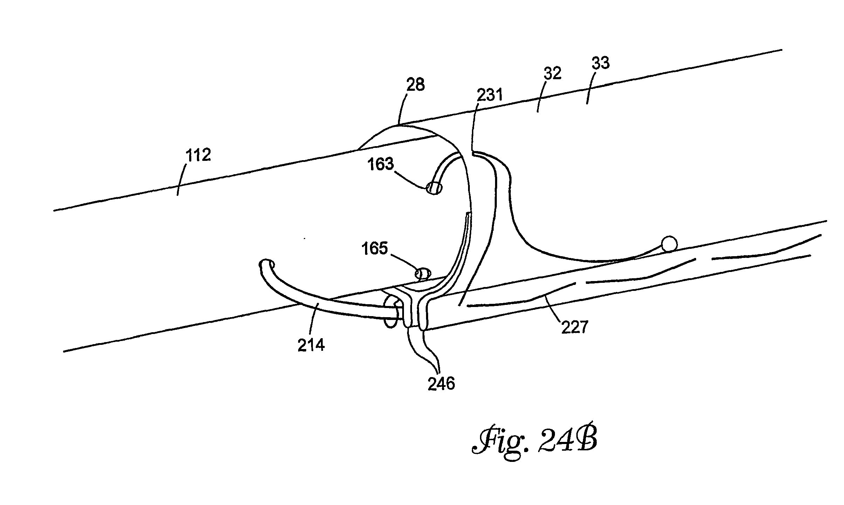

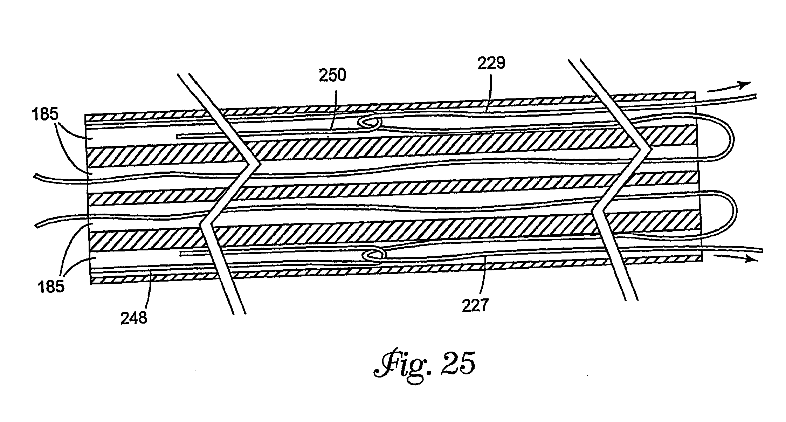

Proximal shaft tube 172 preferably is a multi lumen single extrusion tube, preferably fabricated from PEBAX™ 7233. FIG. 20 is a view in cross section of the delivery system along line a-a in FIG. 19, near the distal end of the proximal shaft tube. The components in this view include sheath 114, proximal shaft tube 172, proximal region of saline extension tube 160, inner shaft 170, two monofilament lines 213 and 214, and main core 202 of retrograde guide wire 200. Proximal shaft tube 172 has central lumen 180 that contains inner shaft 170, monofilament lines 213 and 214, and retrograde guide wire 200 (as well as the proximal portion of saline delivery extension tube 160, within which it is bonded). Central lumen 180 carries the chilled saline from connector 122 at the proximal end of handle 116 and into saline lumen extension tube 160. A series of ports emerging from the saline delivery extension tube 160, not shown, allow the saline to emerge and flow past the contracted bifurcated prosthesis prior to deployment. The proximal shaft tube also contains planetary lumens 185. Preferably there are six planetary lumens. Two of the planetary lumens contain two suture ties 227 and 229 that are used in the delivery and expansion steps for the main branch and side branch of the bifurcated prosthesis as shown in FIGS. 8 and 9 and described in more detail below. Two other planetary lumens are used to contain two tension control threads which work cooperatively with the two tie sutures during expansion of the bifurcated prosthesis, also described in more detail below. These sutures and threads are illustrated in FIGS. 24A, 24B, and 25. The inner shaft has three lumens as described above, as shown in FIG. 20.

### Prosthesis Loading and Deployment Mechanism

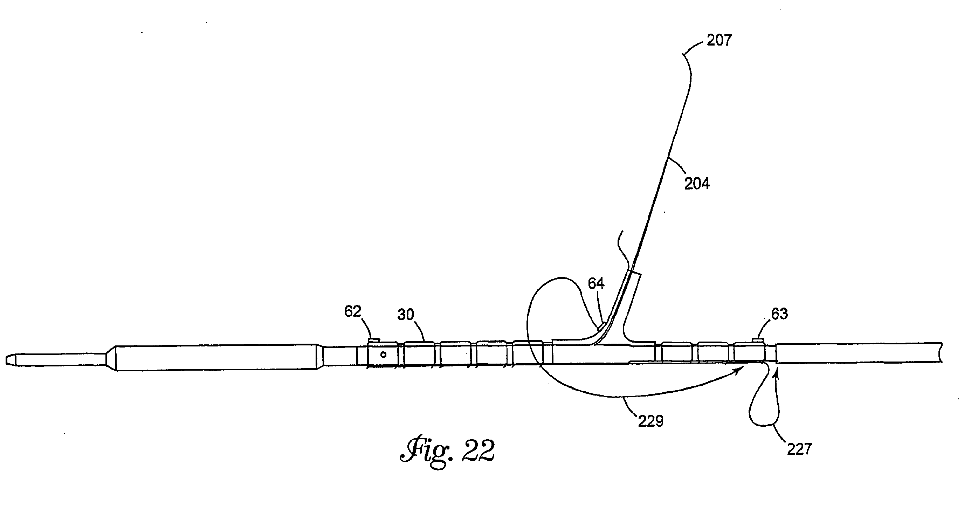

The bifurcated prosthesis is contracted to place it in a low profile condition, such that the sheath will fit over the exterior. The side arm and the curved portion of the retrograde guide wire are bent to a position next to the shaft of the inner catheter prior to the main sheath covering the exterior. The bifurcated prosthesis is loaded into the delivery catheter and positioned around the saline delivery extension tube. As best seen in FIG. 22 the retrograde guide wire emerges from the saline delivery extension tube and through the lumen of the side branch.

FIGS. 24A and 24B, for example, illustrate how the main branch of the bifurcated prosthesis is contracted to attain a low profile condition. Since the stents and the graft material are sutured together at points near the top and bottom of the figure 24A, only the graft material along the sides of the stents can be manipulated and folded. As the graft material is gathered, it is folded in two "S" shaped folds 244, one on each side, as indicated, and extended downward somewhat. A monofilament line 214 is positioned next to, and runs the length of, the downward fold 246, and a suture tie 227 is sewn across the downward fold and surrounds the monofilament line, as indicated in FIGS. 24A and 24B. This suture tie engagement with the monofilament line maintains the graft in a folded condition. As best seen in FIG. 24B a series of sequential stitches from a single suture line are placed along the full length of the downward fold, penetrating the downward fold 246 and surrounding the monofilament line. Preferably, the suture ties progress from the proximal end of the main branch to the distal end of the main branch. At the distal end of the main branch, the suture line engages with the inner catheter 112 in a manner which prevents possible longitudinal movement of the contracted bifurcated prosthesis relative to the inner catheter during sheath retraction.

FIG. 24B depicts distal end 28 of main branch 32 of the bifurcated prosthesis 30 as it is loaded on to the inner catheter 112. Monofilament 214 extends along the back side of the downward folds of the graft material, and extends inside saline delivery extension tube 160. Along the front side of the folds is main branch suture tie 227, with periodic penetrations that encircle monofilament 214. After the distal most penetration through the folds to engage monofilament 214, suture 227 is brought back to the side to encircle a portion of the graft material 33 before it penetrates into a first location 231 on the graft material, then into one port 163 in saline delivery extension tube 160 and out a second hole 165, then through the graft material in a second location 233, then encircles the monofilament 223 before it is doubled back along the same path, until it re-emerges from the graft at the first location 231. The free end of suture tie 227 is then loosely intertwined with one or more of the suture loops adjacent the downward fold.

It is important that at the distal end the suture tie mechanically connects the contracted prosthesis with the inner catheter. This connection serves to maintain the position of the contracted prosthesis during the sheath withdrawal step. Without this connection, the distal end of the main branch could axially compress or "bunch up" during sheath withdrawal, possibly leading to kinking.

The end of the monofilament running along the outside of the downward fold on the main branch is at the proximal end of the main branch. The monofilament extends distally along the full length of the main branch. At the distal end of the main branch, the monofilament extends into the inside of the saline delivery extension tube and from there it extends proximally along the outside of the inner shaft as indicated in FIG. 20. The other end of the monofilament for the main branch terminates at release knob 138, which is releasably engaged with the proximal end of handle mechanism 116, as shown in FIG. 17.

The side branch of the bifurcated prosthesis is similarly contracted about the retrograde guide wire. Second monofilament line 213 extends along the gathered and folded graft material of the side branch. This side branch fold extends on the side opposite of the arm stop. A second suture tie 229 is sequentially sewn across the fold of the side branch and surrounds the second monofilament along the full length of the side branch. Second monofilament 213 has a first end near the end of the contracted side branch, and extends towards the juncture of the side branch with the main branch. At this point, second monofilament 213 penetrates the wall of the graft material of the main branch, and extends through a passageway to the inside of the saline delivery extension tube. It then extends proximally through the same lumen as first monofilament 214, as indicated in FIG. 20, to second release knob 139 on the proximal end of the handle mechanism.

Suture tie 229 for side branch 34 progresses from its proximal end, and a series of stitches is placed progressively towards end 40 of side branch 34, terminating in a tight wrap about the distal most end 40 of the compressed and folded side branch. Any excess length of suture material for the side branch is brought back and tucked in the prior stitches. The other end of the suture tie 229 for the side branch is positioned along the exterior of the proximal portion of the contracted main branch, and positioned within one of the planetary lumens (185) of proximal shaft tube 172. Likewise, the proximal end of suture tie 227 for the main branch is positioned within another of the planetary lumens of proximal shaft tube 172. Both of these suture ties extend fully through the length of the delivery catheter, and terminate at the same knob as their respective monofilament lines. In other words, the side branch has a monofilament line and a suture tie line that extend proximally to a release knob 139, and the main branch likewise has a monofilament line and a suture tie line extending proximally to a different release knob 138.

Once the bifurcated prosthesis is loaded and secured onto the inner catheter, the side branch is folded back and the sheath is positioned over the bifurcated prosthesis. The delivery catheter and prosthesis are now in a position to be delivered into the vasculature.

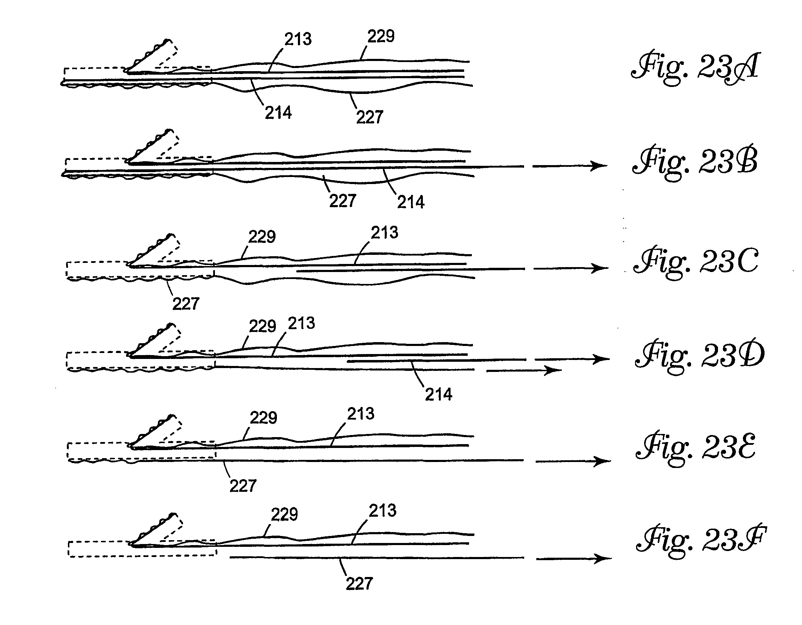

FIGS. 22 and 23A to 23F depict the deployment mechanism after the contracted bifurcated prosthesis has been positioned at the iliac bifurcation. The prosthesis is represented by the dotted line. Two monofilament lines 213 and 214 run along the side branch and main branch, respectively. The monofilaments extend along the lengths of the branches and then "double-back" proximally. Suture stitch lines 227 and 229 engage monofilaments 213 and 214, and meander along the lengths of the branches, then traverse proximally. FIG. 23A shows the monofilament lines and the suture lines placement before expansion of the bifurcated prosthesis, such as shown in FIG. 7. FIG. 23B indicates the first step in the expansion of the main branch. Release knob 139 connects to monofilament 213. Release knob 139 is retracted away from handle mechanism 116, pulling on the monofilament line. The other end of the monofilament begins to slide distally. It pulls through the engagements of the suture tie. FIG. 23C shows the monofilament has become completely disengaged from the suture tie, and continues to move proximally. The suture tie, which is also connected to the release knob now begins to be tensioned (FIG. 23D). The suture tie is intentionally extra long, having sufficient suture to allow for the monofilament line to become completely disengaged from the main branch prior to the suture tie beginning to pull free. As the suture tie is pulled further, the stitches through the folds of the main branch graft material begin to pull free of the graft material, beginning from the proximal end (FIG. 23E). As the stitches disengage (and the chilled saline is shut off), the proximal end of the main branch begins to expand under the forces of the support stents. Continued pulling on the suture tie away from the handle mechanism completely frees the suture tie from the main branch, as shown in FIG. 23F.

The side branch is expanded via the same mechanism, with initial pulling on the monofilament associated with the side branch. After the monofilament slides free of the suture tie stitches, the suture tie is then pulled, freeing the stitching from the folds of the graft material of the side branch, and allowing the stents to expand, once warmed to body temperature.

The excess length of suture tie material is in planetary lumen 185 of proximal shaft tube 172. It is placed there from the proximal end (see FIGS. 18A and 18C). In order to prevent the excess suture tie material from inadvertently pulling out from the planetary lumen prematurely, a tensioning mechanism is utilized. This is depicted in FIG. 25. Four of the planetary lumens of the proximal shaft tube are shown. In two of them run the suture ties from the branches of the bifurcated prosthesis. These run the full length of the tube, and the excess length brought into the proximal end of an adjacent planetary lumen. The proximal ends of the suture ties then traverse to their respective release knobs 138 and 139 on handle mechanism 116. At the distal ends of the lumens containing the excess length of suture tie are the tensioning threads. These threads, also of suture material, are secured at the distal end of the proximal shaft tube. These threads traverse towards the proximal ends of the lumens, and are then "doubled back" in the same lumen. The tension threads are looped through the loops on the suture ties. As the suture ties are pulled, they pull back on the tensioning threads, thereby emerging from the proximal end of the planetary lumens in a controlled manner.

## Claims

1. A delivery catheter having a distal end for insertion in a patient's vasculature for delivering a prosthesis comprising:
an inner catheter (112) over which the prosthesis is loaded;
a retrograde guide wire (200) having a main core (202) and a retrograde core (204), the retrograde core (204) having a first end joined to the main core and a second end; and
a handle (116) at a proximal end of the delivery catheter; **characterised in that** the handle (116) has a first control element for advancing the main core (202) in a distal direction or retracting the main core (202) in a proximal direction, wherein the second end (207) of the retrograde core (204) is curved such that retraction of the main core (202) causes the second end (207) of the retrograde core (204) to move away from the main core (202).

2. The delivery catheter of claim 1 further comprising a retractable outer sheath (114) defining a lumen through which the inner catheter (112) extends and further defining a space (130) between the inner catheter (112) and the sheath (114) configured to contain the prosthesis.

3. The delivery catheter of claim 1, wherein the delivery catheter is for delivering a bifurcated prosthesis through a patient's vasculature to the bifurcation of a first vessel into second and third vessels, the bifurcated prosthesis having a main branch defining a first lumen and having first and second ends adapted for placement in the first and second vessels, respectively, and a side branch (34) defining a second lumen and having a first end fluidly connected to the main branch and a second end adapted for placement in the third vessel, wherein:
the retrograde guide wire is configured such that the retrograde core (204) is adapted to extend through the lumen of the side branch (34) of the bifurcated prosthesis when the prosthesis is loaded on the delivery catheter; and
the first control element is adapted for changing the position of the retrograde guide wire (200) with respect to the inner catheter (112).

4. The delivery catheter of claim 3 further comprising an outer sheath (114) defining a lumen through which the inner catheter (112) extends and further defining a space (130) between the inner catheter (112) and the sheath (114) in which the bifurcated prosthesis is loaded.

5. The delivery catheter of claim 2 or 4, wherein the handle (116) further comprises a second control element configured for retracting the outer sheath (114).

6. A prosthesis delivery system comprising the delivery catheter of claim 3 and a bifurcated prosthesis which is provided with a first engaging member (224) adjacent the second end and the retrograde core (204) further comprises a second engaging member (211), the second engaging member (211) being configured to engage the first engaging member (224) when the retrograde guide wire (200) is retracted such that the second end of the side branch is moved away from the first end of the side branch.

7. The delivery catheter or system of any one of the preceding claims, wherein the distal most portion of the main core (202) of the retrograde guidewire (200) is attached side-by-side with the proximal end of the retrograde core (204) of the retrograde guidewire (200) by a swaged and soldered connector tube (206).

## Patentansprüche

1. Zuführungskatheter mit einem distalen Ende zur Einführung in das Gefäßsystem eines Patienten zum Zuführen einer Prothese, wobei der Zuführungskatheter Folgendes umfasst:
einen inneren Katheter (112), auf welchen die Prothese geladen ist;
einen retrograden Führungsdraht (200) mit einem Hauptkern (202) und einem retrograden Kern (204), wobei der retrograde Kern (204) ein erstes Ende, welches mit dem Hauptkern verbunden ist, und ein zweites Ende aufweist; und
einen Griff (116) an einem proximalen Ende des Zuführungskatheters; **dadurch gekennzeichnet, dass** der Griff (116) ein erstes Steuerungselement zum Vorschieben des Hauptkerns (202) in eine distale Richtung oder zum Zurückziehen des Hauptkerns (202) in eine proximale Richtung aufweist, wobei das zweite Ende (207) des retrograden Kerns (204) gekrümmt ist, so dass ein Zurückziehen des Hauptkerns (202) bewirkt, dass das zweite Ende (207) des retrograden Kerns (204) sich von dem Hauptkern (202) wegbewegt.

2. Zuführungskatheter nach Anspruch 1, welcher weiter eine zurückziehbare äußere Hülle (114) umfasst, welche ein Lumen definiert, durch welches sich der innere Katheter (112) erstreckt, und welche weiter einen Raum (130) zwischen dem inneren Katheter (112) und der Hülle (114) definiert, welcher zum Enthalten der Prothese eingerichtet ist.

3. Zuführungskatheter nach Anspruch 1, wobei der Zuführungskatheter zum Zuführen einer gabelförmigen Prothese an die Verzweigung eines ersten Gefäßes in ein zweites und ein drittes Gefäß durch ein Gefäßsystem eines Patienten geeignet ist, wobei die gabelförmige Prothese einen Hauptast, welcher ein erstes Lumen definiert und ein erstes und ein zweites Ende besitzt, welches für eine Platzierung in dem ersten bzw. dem zweiten Gefäß eingerichtet ist, und einen Seitenast (34), welcher ein zweites Lumen definiert und ein erstes Ende in Fluidverbindung mit dem Hauptast und ein zweites Ende, welches zur Platzierung in dem dritten Gefäß eingerichtet ist, besitzt, aufweist, wobei:
der retrograde Führungsdraht so gestaltet ist, dass der retrograde Kern (204) eingerichtet ist, um sich durch das Lumen des Seitenastes (34) der gabelförmigen Prothese zu erstrecken, wenn die Prothese auf den Zuführungskatheter geladen ist; und
das erste Steuerungselement eingerichtet ist, um die Position des retrograden Führungsdrahts (200) bezüglich des inneren Katheters (112) zu verändern.

4. Zuführungskatheter nach Anspruch 3, welcher weiter eine äußere Hülle (114) umfasst, welche ein Lumen definiert, durch welches sich der innere Katheter (112) erstreckt, und weiter einen Raum (130) zwischen dem inneren Katheter (112) und der Hülle (114) definiert, in welchen die gabelförmige Prothese geladen ist.

5. Zuführungskatheter nach Anspruch 2 oder 4, wobei der Griff (116) weiter ein zweites Steuerungselement umfasst, welches für ein Zurückziehen der äußeren Hülle (114) eingerichtet ist.

6. Prothesenzuführungssystem, welches den Zuführungskatheter nach Anspruch 3 und eine gabelförmige Prothese, welche mit einem ersten Kopplungselement (224) neben dem zweiten Ende versehen ist, umfaßt, wobei der retrograde Kern (204) weiter ein zweites Kopplungselement (211) umfasst, wobei das zweite Kopplungselement (211) eingerichtet ist, um das erste Kopplungselement (224) einzukoppeln, wenn der retrograde Führungsdraht (200) zurückgezogen ist, so dass das zweite Ende des Seitenastes von dem ersten Ende des Seitenastes wegbewegt ist.

7. Zuführungskatheter oder System nach einem der vorhergehenden Ansprüche, wobei der distalste Teilbereich des Hauptkerns (202) des retrograden Führungsdrahts (200) durch eine geschmiedete und gelötete Verbindungsröhre (205) sich neben dem proximalen Ende des retrograden Kerns (204) des retrograden Führungsdrahts (200) befindend angebracht ist.

## Revendications

1. Cathéter de mise en place ayant une extrémité distale destinée à être insérée dans le système vasculaire d'un patient pour la mise en place d'une prothèse, qui comprend :
■ un cathéter interne (112) sur lequel la prothèse est chargée ;
■ un fil guide rétrograde (200) ayant une âme principale (202) et une âme rétrograde (204), l'âme rétrograde (204) ayant une première extrémité jointe à l'âme principale et une seconde extrémité ; et,
■ une poignée (116) au niveau d'une extrémité proximale du cathéter de mise en place, **caractérisé en ce que** la poignée (116) a un premier élément de commande destiné à faire progresser l'âme principale (202) dans une direction distale ou à rétracter l'âme principale (202) dans une direction proximale, la seconde extrémité (207) de l'âme rétrograde (204) étant incurvée de manière à ce que la rétraction de l'âme principale (202) entraîne l'éloignement de la seconde extrémité (207) de l'âme rétrograde (202) par rapport à l'âme principale (202).

2. Cathéter de mise en place selon la revendication 1, qui comprend en outre une gaine extérieure rétractable (114) définissant une lumière à travers laquelle le cathéter intérieur (112) s'étend et définissant en outre un espace (130) entre le cathéter intérieur (112) et la gaine (114) configuré pour contenir la prothèse.

3. Cathéter de mise en place selon la revendication 1, dans lequel le cathéter de mise en place est destiné à mettre en place une prothèse bifurquée à travers le système vasculaire d'un patient jusqu'à la bifurcation d'un premier vaisseau en des deuxième et troisième vaisseaux, la prothèse bifurquée ayant une ramification principale définissant une première lumière et ayant des première et seconde extrémités adaptés à être positionnées dans les premier et second vaisseaux, respectivement, et une ramification latérale (34) définissant une seconde lumière et ayant une première extrémité en connexion fluidique avec la ramification principale et une seconde extrémité adaptée à être positionnée dans le troisième vaisseau, dans lequel :
■ le fil-guide rétrograde est configuré de manière à ce que l'âme rétrograde (204) soit adaptée à s'étendre dans la lumière de la ramification latérale (34) de la prothèse bifurquée lorsque la prothèse est positionnée sur le cathéter de mise en place ; et,
■ le premier élément de commande est adapté à modifier la position du fil-guide rétrograde (200) relativement au cathéter interne (112).

4. Cathéter de mise en place selon la revendication 3, qui comprend en outre une gaine extérieure (114) définissant une lumière à travers laquelle le cathéter intérieur (112) s'étend et définissant en outre un espace (130) entre le cathéter interne (112) et la gaine (114) dans lequel la prothèse bifurquée est chargée.

5. Cathéter de mise en place selon la revendication 2 ou 4, dans lequel la poignée (116) comprend en outre un second élément de commande configuré pour rétracter la gaine extérieure (114).

6. Système de mise en place de prothèse qui comprend le cathéter de mise en place selon la revendication 3 et une prothèse bifurquée qui est dotée d'un premier élément de mise en prise (224) adjacent à la seconde extrémité et l'âme rétrograde (204) comprend en outre un second élément de mise en prise (211), le second élément de mise en prise (211) étant configuré pour se mettre en prise avec le premier élément de mise en prise (224) lorsque le fil-guide rétrograde (200) est rétracté de manière à ce que la seconde extrémité de la ramification latérale soit déplacée de la première extrémité de la ramification latérale.

7. Système ou cathéter de mise en place selon l'une quelconque des revendications précédentes, dans lequel la partie la plus distale de l'âme principale (202) du fil-guide rétrograde (200) est fixée à côté de l'extrémité proximale de l'âme rétrograde (204) du fil-guide rétrograde (200) par un tube connecteur estampé et soudé (206).
